# EUROPEAN PATENT APPLICATION

(11) **EP 3 001 911 A1**
(43) Date of publication of application: **06.04.2016**
(21) Application number: 14382380.5
(22) Date of filing: 03.10.2014
(51) Int. Cl.: A23K 10/40, A61K 36/28

(54) **Composition with stevia rebaudiana bertoni and uses thereof**

(71) Applicant: Biolittletec, S.L., 08010 Barcelona (ES)
(72) Inventor: Sánchez-Lafuente Mariol, Luis, 08034 Barcelona (ES); González Rubio, Judith, 08902 L'Hospitalet de Llobregat (ES)
(74) Representative: ZBM Patents ApS

(57) **Abstract**

A composition based on a dried part of the *Stevia rebaudiana bertoni* plant for use to prevent and/or treat at least one animal abnormal behaviour. An edible product comprising said composition, and at least one edible acceptable ingredient for use in the prevention and/or treatment of at least one animal abnormal behaviour.

## Description

The present invention relates to the use of a composition of *Stevia rebaudiana bertoni* which is in the benefit of the animals themselves, of the animals' manipulators and of the final consumers of the animals or animal related products.

### BACKGROUND ART

There is public concern not only on the quality of the food products but also on the way the products are obtained. In other words, there is an increase societal awareness that animal production matters, but also the production methods.

For animal products, the conditions in which animals are reared and slaughtered are of prime importance. In connection with this the animal welfare is an essential issue in modern farming systems due to the animal perceived mismatch between animals' actual environments and their natural habitats. Animal welfare arises from the acceptance that animals are sentient beings. Welfare problems can be caused by the physical environment (e.g., poor housing and lack of food) and diseases, but also by "purely psychological" factors that affect the functioning of the animals.

Those factors affecting animal welfare related with the physical environment can be controlled by the livestock producer only to some extent. For instance in an intensive production it is difficult to control all the so called adverse situations, such as the limited space available for the animal, insufficient access to the feeder, drinking and fodder trough, reduced rest area or adverse environmental stimuli. In addition, the social aggression and/or territorial behaviour in order to establish a social dominance and a hierarchy in a housed herd are inherent to the animal behaviour and are mostly out of the control of the livestock producer.

All the above factors interact with each other, affecting the animal's health both physically and psychologically. The consequences of low animal welfare are increased probability of clinical disease, of behaviour abnormalities, more pronounced sickness behaviour, and eventually a reduction in the quality of animal products.

A poor animal welfare is manifested by many different signs. Among all of them the so called animal abnormal behaviours are especially relevant. It is also widely accepted that there are significant relationships between the behavioural reactivity of the animals and their sociability, as well as their manipulation by the farmers and personnel in charge. The prevention and/or treatment of animal abnormal behaviours, do not only revert on the animal welfare, and quality of the animal products, but it also improves animal sociability and ameliorates and facilitates the handling practices by the farmer, reducing the farmer's risk to be injured during the manipulation of the herd.

Consequently, the concept of high animal welfare includes the prevention and/or treatment of abnormal behaviours. Eventually, the prevention and/or treatment of abnormal behaviours assure good sociability and improved animals' manipulation, and it also involves increasing the animal products' quality. The quality of animal products has important economic consequences, representing huge yearly economic losses for animal producers and animal products dealers.

In general, current activities to promote high animal welfare, and hence, to avoid and/or treat an animal abnormal behaviour, mainly focus, on the one hand, on animal living conditions, including facilities and equipment (da Costa, M.J.R.P. *et al.* Strategies to promote farm animal welfare in Latin America and their effects on carcass and meat quality traits. Meet Sci. 2012, Vol. 92, No. 3, pages 221-6; Veissier, I. et al. Animal welfare: A result of animal background and perception of its environment. Animal frontiers. 2012, Vol. 2, No. 3, pages 7-15). Additionally, they also focus on animal handling procedures (Langley, R.L. and Morrow, W.E., Livestock handling-minimizing worker injuries. J. Agromedicine. 2010, Vol. 15, No. 3, pages 226-35). The limitations of said activities are that the conditions can be controlled and improved up to certain extend, for the reasons above explained.

There is another line of actuation based on genetic selection of welfare-related traits, since they are at least partially under genetic control (Beaumont, C. et al. The European experience in poultry welfare-A decade ahead. Poultry Science. 2012, Vol. 89, pages 825-31). However, this last line has also important limitations and is extremely complicated to put into practice due to the fact that the so called welfare-related traits are complex genetic traits, and also due to the genetic determinism of such complex traits which is strictly dependent on environmental conditions.

In view of the above mentioned limitations in the currently methods applied to treat and/or prevent animal abnormal behaviours, there is a permanent and increasing interest and need of compositions that comply with the regulatory provisions for their prevention and/or treatment.

### SUMMARY OF THE INVENTION

Inventors have surprisingly found that a composition based on a dried part of the *Stevia rebaudiana bertoni* plant, disclosed herein has the effect of preventing and/or treating a specific manifestation of poor animal welfare, namely, animal abnormal behaviours.

To the best of the inventors' knowledge the use of a composition based on a dried part of *Stevia rebaudiana bertoni* has neither been suggested to influence animal abnormal behaviours, and also as a consequence to indirectly affect animal products in terms of quality.

In the context of the present invention the expression "non-human animal" includes livestock, working animals, sport animals, laboratory animals, animals present in a zoo and domestic or companion animals, including exotic companion animals.

In the context of the present invention, the expression "animal product" means any material derived from the body of a non-human animal intended for human and non-human consumption. Examples of animal products without limitation are fat, flesh, blood, milk, eggs, fur and lesser known products such as isinglass and rennet.

Since the composition of the present invention is a natural product, it is safe for the animals and allows the treatment and/or prevention of animal abnormal behaviours by means of natural ingredients, which may eventually be incorporated in the common food/fodder/feed/diet of the animals. In the context of the present invention, the terms "food", "fodder", "feed" and "diet" are used indistinctly to refer to any foodstuff used specifically to feed animals, as well as the corresponding food or feed premix.

In the context of the present invention, the expression "livestock" refers to domesticated animals raised in an agricultural setting to produce commodities such as food, fibre and labour, including poultry or farmed fish.

In the context of the present invention, the expressions "pet" and "companion animal" are used indistinctly to refer to an animal kept primarily for a person's company or protection.

Document WO 2009071277 A1 relates to a composition comprising *Stevia* extract or its constituents to improve cognitive functions, such as learning, memory, and alertness, as well as relieving psychological pressure, in general. This document discloses the use of an extract of *Stevia* made from any species of the genus *Stevia,* including *Stevia rebaudiana.* The term "extract" as used in said document relates to an extract from the whole plant using a solvent. Documents Shiozaki, K. et al. (Inhibitory effects of hot water extract of the Stevia stem on the contractile response of the smooth muscle of the guinea pig ileum. Biosci. Biotechnol. Biochem. 2006, Vol. 70, pages 489-494) and Matera, S. et al. (Sedative and antispasmodic effects of Stevia rebaudiana and non-competitive inhibition of intestinal contractility by stevioside. Pharmacology on line. 2012, Vol. 2, No. 1, pages 1-8) disclose the sedative and antispasmodic effects, and the inhibition of the contractile response of the smooth muscle of *Stevia rebaudiana,* respectively.

Document WO 2009/071277 A1 supports the disclosed effects on a rodent model of learning and memory and laboratory mice. Similarly, Shiozaki, K. *et al.* (2006, *supra*) and Matera, S. *et al.* (2012, *supra*) supports the intestinal effects of the *Stevia* extract observed therein using Male Hartley guinea pigs and Sprague-Dawley rats, respectively. In summary, said documents do neither mention nor suggest that using dried parts of specifically the *rebaudiana bertoni* species of *Stevia,* animal abnormal behaviours are prevented and/or treated.

In contrast, the present invention discloses the beneficial effects of the herein disclosed compositions and edible products on animal abnormal behaviours, in general. Moreover, the inventors have surprisingly observed the herein disclosed effects in many different animals, including pets and livestock, under real living animal conditions, i.e. intensive productivity conditions. All the previously mentioned state of the art documents are completely silent regarding the benefits of their compositions on animal abnormal behaviours, on which the compositions of the present invention are fully effective.

Document EP 2457450 A1 discloses an edible composition having an amount of steviol glycosides comprised between 150 ppm and 3,000 ppm. The composition comprises a mixture of dried solids that is a mixture of the leaves and stems of the *Stevia rebaudiana* plant, the leaves and stems being in a ratio comprised between 25:75 and 35:65 w/w %. It discloses its use as egg colouring agent and as egg shell hardening agent. The document suggests that said effects are due to an improvement in the health of the hens producing the eggs, due to a reduction of the stress of the hens during the productive system. The document also teaches the use of the edible composition disclosed therein as an animal productivity enhancer.

It is widely known that the biochemical mechanisms underlying the response of an animal to stress have nothing to do with those involved in an animal abnormal behaviour, and subsequently in animal sociability. As result of animal stress, there is an activation of both the sympathetic nervous system and the adrenal medulla, which involves the release of catecholamines (epinephrine, norepinephrine and dopamine); and also an activation of the hypothalamus-pituitary-adrenal cortex system, which involves the release of glucocorticoids (cortisone, cortisol and corticosterone).

In contrast, animal abnormal behaviours results mainly from an alteration of the serotonergic system. For instance, it is well known that at the neurochemical level, the main substance involved in animal aggression, as a manifestation of animal abnormal behaviours is the serotonin.

In view of the above common general knowledge, and the teachings of document EP 2457450 A1, the skilled person would be oriented to use the composition disclosed therein in order to improve properties of the eggs, but not to prevent and/or treat animal abnormal behaviours, in general. Consequently, a first aspect of the present invention relates to a composition based on a dried part of *Stevia rebaudiana bertoni* for use in the prevention and/or treatment of at least one animal abnormal behaviour.

*Stevia rebaudiana bertoni* is one of the 240 species of the genus *Stevia* in the family of *Asteraceae. Stevia rebaudiana bertoni* is a plant from South America tropical origin extensively cultivated around the world (Australia, Canada, India, Mexico, Brazil, Paraguay, Bolivia, etc.), and mainly marketed in Paraguay. *Stevia rebaudiana* is, hence, widely cultivated.

### DETAILED DESCRIPTION OF THE INVENTION

As detailed above, the present invention relates to a composition based on a dried part of *Stevia rebaudiana bertoni* for use in the prevention and/or treatment of at least one animal abnormal behaviour.

This aspect can be alternatively formulated as the use of a dried part of the *Stevia rebaudiana bertoni* plant for the manufacture of a composition to prevent and/or treat at least one animal abnormal behaviour. Similarly, this may be also alternatively formulated as a method for the prevention and/or treatment of at least one animal abnormal behavior, comprising administering to said animal in need thereof an effective amount of any of the compositions of the present invention.

The term "effective amount" as used herein, means an amount of an active agent high enough to deliver the desired benefit, but low enough to avoid serious side effects within the scope of medical judgment.

In a particular embodiment, the plant of *Stevia rebaudiana bertoni* used is from Paraguay. In a still more particular embodiment, the plant of *Stevia rebaudiana bertoni* used is from the Amambay mountain chain in Paraguay.

In a particular embodiment, the animal is a non-human animal. In a still more particular embodiment, the non-human animal selected from the group consisting of: livestock animal and companion animal. In a still more particular embodiment, the non-human animal is selected from the group consisting of: chicken, beef, pig, sheep, cow, hen, horse, donkey, pony, turkey, rabbit, quail, partridge, goat, pheasant, ostrich, duck, dog, cat, an exotic domestic animal and a zoo animal.

The composition of the present invention is preferably obtained by a process comprising the following steps: a) drying the plant *Stevia rebaudiana bertoni* at a temperature comprised between 18 °C and 35 °C and, b) separating the leaves from the stems to obtain a dried leaves fraction and a dried stems fraction

Accordingly, in a particular embodiment of the present invention, the composition is based on the dried stems fraction. In another particular embodiment of the present invention, the composition is based on the dried leaves fraction.

In another particular embodiment, the composition of the invention is obtainable by a process comprising the above steps a) and b) and additionally, comprising a step c), of mixing the dried leaves and the dried stems fractions of the *Stevia rebaudiana bertoni* plant obtained in step b). In still another particular embodiment, the dried leaves and the dried stems fractions of the *Stevia rebaudiana bertoni* plant obtained in step b) are mixed in a ratio comprised between 25:75 and 35:65 w/w %, to obtain a mixture of dried solids. In a still more particular embodiment, the ratio is 30:70. Abbreviation w/w % means ratio of percentage by weight.

Accordingly, in a particular embodiment of the present invention, the composition is based on a mixture of the dried leaves and the dried stems fractions of step c). In a particular embodiment, said mixture has a ratio of dried leaves and dried stems comprised between 25:75 and 35:65 w/w %.

In other particular embodiments, the dried stems fraction or the dried leaves fraction or any of the above mixtures of the dried leaves and the dried stems fraction is a solid powder. Accordingly, in other particular embodiments, the above process of the present invention further comprises a step d), of grinding the dried leaves fraction, or grinding dried stems fraction, or grinding both the dried leaves and the dried stems fractions of step c), to obtain a powder with the desired particle size average. In a more particular embodiment, the particle size of any of the powders of the invention is between 10 µm and 10.0 mm. Still more particular ranges are from 10 µm to 5.0 mm.

In the context of the present invention, the expression "based on" is to be understood as encompassing all the possible formulations which, either contains a dried part of the plant *Stevia rebaudiana bertoni* as such, or an extract obtained from it, namely, and without limitation, a solution, emulsion or suspension, such as beverages, pastes and oily suspensions. Throughout the specification and claims, the term "extract" is intended to be used broadly, encompassing extracts made by conventional means, acceptable for use for animal and/or human consumption. Thus the solvent to be employed for its preparation should be approved by the various regulatory agencies for the intended use.

As already stated in the previous section, the composition according to the invention is also suitable to be used as an edible product. Thus, a second aspect of the present invention is an edible product comprising an effective amount of any of the compositions of the present invention for use to prevent and/or treat at least one abnormal behaviour. In a particular embodiment, the edible product comprises an effective amount of a solid mixture of the dried leaves and dried stems of *Stevia rebaudiana bertoni,* and at least one edible acceptable ingredient.

The above edible product may be obtained by the process as defined above, further comprising the step of mixing the dried leaves or the dried stems fractions of step b), or the mixture of step c) or the solid powder of step d) with at least one edible acceptable ingredient to form an edible product. The composition of the invention in the forms described above is in an amount, in respect of the edible product, between 0.014 and 0.5 w/w %. In another particular embodiment, the amount is between 0.028 and 0.5 w/w %. In another particular embodiment, the amount is between 0.014 and 0.2 w/w %, which is preferably used for the prevention of animal abnormal behaviours. In another particular embodiment, the amount is between 0.2 and 0.3 w/w %, which is preferably used for the treatment of animal abnormal behaviours.

In another particular embodiment, the final concentration of steviol glycosides comprised in the edible product of the invention is between 75 parts per million (ppm) and 3.000 ppm with respect to the total weight of the edible composition. As the amount of steviol glycosides in the edible product must be comprised between 75 ppm and 3.000 ppm, the edible product comprises the appropriate amount of the dried parts of *Stevia rebaudiana bertoni* that yields the amount of steviol glycoside previously indicated.

By way of examples, and without limitation, particular embodiments are indicated below. It is indicated: (i) the amount of the composition of the present invention based on: (1) mixing the dried stems and dried leaves fractions of *Stevia rebaudiana bertoni*; or (2) only the dried leaves fraction; or (3) only the dried stems fractions, which is added to 1,000 kg of e.g. an animal fodder; as well as the resulting proportion of steviol glycosides in the final edible product (in brackets):
- mixing (1) 98 grams (g) of stems fraction and 42 g of leaves fraction; (2) alternatively, 74 g of leaves fraction; (3) alternatively, 222 g of stems fraction (resulting in all cases in 75 ppm of steviol glycosides)
- mixing (1) 196 g of stems fraction and 84 g of leaves fraction; (2) alternatively, 149 g of leaves fraction; (3) alternatively, 447 g of stems fraction (resulting in all cases in 150 ppm of steviol glycosides)
- mixing (1) 393 g of stems fraction and 167 g of leaves fraction; (2) alternatively, 298 g of leaves fraction; (3) alternatively, 894 g of stems fraction (resulting in all cases in 300 ppm of steviol glycosides)
- mixing (1) 785 g of stems fraction and 335 g of leaves fraction; (2) alternatively, 596 g of leaves fraction; (3) alternatively, 1,788 g of stems fraction (resulting in all cases in 600 ppm of steviol glycosides)
- mixing (1) 1,962 g of stems fraction and 838 g of leaves fraction; (2) alternatively, 1,492 g of leaves fraction; (3) alternatively, 4,476 g of stems fraction (resulting in all cases in 1,500 ppm of steviol glycosides)
- mixing (1) 3,920 g of stems fraction and 1,680 g of leaves fraction; (2) alternatively, 2986 g of leaves fraction; (3) alternatively, 8,958 g of stems fraction (resulting in all cases in 3,000 ppm of steviol glycosides)

In the sense of the invention, the expression an "amount of steviol glycosides" means mixtures of steviol glycosides, namely mixtures of stevioside, rebaudiosides A, B, C, D, E and F, dulcoside A, steviolbioside, and rubusoside. As it is well known in the art, the diterpene known as steviol is the aglycone of stevia's glycosides, which are constructed by replacing steviol's carboxyl hydrogen atom (at the bottom left of the compound of formula I) with glucose to form an ester, and replacing the hydroxyl hydrogen (at the top of the compound of formula I) with combinations of glucose and rhamnose to form an acetal.

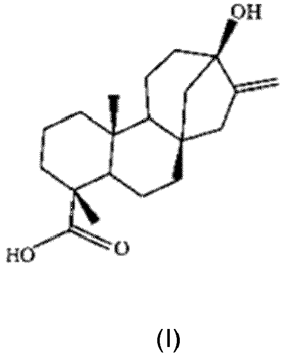

In a particular embodiment the edible composition comprises a mixture of rebaudioside A, stevioside, rebaudioside F, rebaudioside C, dulcoside A, an isolated non-identified compound with molecular weight of 804 g/mol; MW804, rubusoside and rebaudioside B.

The composition of the invention can be included in a variety of edible products. The term "edible product" is used herein in its broadest meaning, including any type of product, in any form of presentation, which can be ingested by an animal, including a human; i.e. a product that is organoleptically acceptable. The edible product includes the proteins, vitamins, fat, salts, amino acids, and cellulose usually present in these types of compositions. Variation of the percentages and components may exist depending on the final consumer. Selection of the edible acceptable ingredient(s) and the most appropriate methods for formulation in view of the particular purpose of the composition is within the scope of ordinary persons skilled in the art of food technology.

The term "edible acceptable ingredient" as used herein pertains to compounds, materials, compositions, and/or dosage forms which are suitable for use in food preparations, such as vitamins, proteins, fibre, carbohydrates, fat, etc., which are widely employed in fodders, nutritional supplements, liquid preparations, etc. Each ingredient must also be "acceptable" in the sense of being compatible with the other ingredients of the formulation.

The edible product of the invention may be formulated as a powder or a liquid (i.e. a liquid infusion of the powder or a solution of the powder) to be used as food additive, or as nutritional supplement, or as fodder. When formulated as a food additive it may be added to any previously processed feed or liquid, such as milk, vitamin-enriched water, fruit juices, and even be mixed with drug formulations. As above indicated, the edible product may also be itself a processed food, in particular a fodder or a nutritional supplement.

The nutritional supplements comprising the edible product of the invention include all those essential ingredients useful for providing the nutrients, such as vitamins, minerals, fibre, fatty acids, or amino acids, that may be missing or may not be consumed in sufficient quantity in a diet. The edible product does not prejudice with the supply of all other components.

In another particular embodiment, the edible product of the invention is a fodder. In such case, the fodder is the mixture containing the edible product of the invention, and including common ingredients of this type of compositions, such as hay, straw, silage, compressed and pelleted feeds, oils and mixed rations, molasses and also sprouted grains and legumes.

In the context of the present invention, the expression "animal abnormal behaviour" covers any undesirable and strange-looking behaviour. In non-human animals, they include stereotype and social instability. A non-human abnormal behaviour is developed in anxious and/or uncomfortable animals in response to conflict situations due to, and without limitation, feed deprivation, movement and activity restriction, frustration and/or impossibility of escaping.

Among the animal abnormal behaviours, the so called "abnormal repetitive behaviour (also known as ARB) is especially relevant.

In the context of the present invention, the expressions "abnormal repetitive behaviour" (or ARB), "stereotypic behaviour" and "stereotype" are used indistinctively. Those expressions define a repetitive, invariable conduct, without apparent function.

Accordingly, in a particular embodiment, all the compositions of the present invention are used to treat and/or prevent animal stereotypes.

Examples of non-human animal stereotypes are, without limitation: (1) oral/oro-nasal ARBs, typically in ungulates, cattle, horses, pigs, sheep and their wild relatives; (2) locomotor ARBs like pacing, typically in carnivores; and (3) self-harm and over-grooming or self-licking/chewing in pet/companion animals, like parrots and cats.

Among oro/nasal ARBs are to be mentioned, and without limitation, bar biting and vacuum chewing. Vacuum chewing and bar biting are typically observed in sows confined in stalls or tether systems.

In the context of the present invention, the expression "vacuum chewing" means an innate, fixed action pattern of animal behaviour that is performed in the absence of external *stimuli*, namely with no substrate in their mouth.

In the context of the present invention, by the expression "bar biting" is to be understood a behaviour pattern wherein the animals open their mouths wide and grasp the rail at head height, moving then their heads from side to side with the rail pressed.

Another common stereotype is self-licking or grooming, typically observed in pets or human companion animals, such as cats. Cats typically spend between 7 and 12 hours per day grooming them to cleanliness. Grooming is a natural reaction of cats. However, sometimes this normal grooming becomes an obsessive repetitive abnormal behaviour, and the cat shows over-grooming. Some of the reasons of said over-grooming are naturally occurring ones, such as food allergies, allergic reactions to internal or external pests (such as fleas), infections, etc. But there are also psychological causes for over-grooming behaviour (known as "psychological over-grooming", in the context of the present invention). The "psychological over-grooming" reaction is more frequent among more sensitive cats, which over-react to environmental changes. The consequence of over-grooming is the appearance of areas without hair which may end in abrading skin surface and eventually serious infections.

In some cases over-grooming is a so called "displacement behaviour". Eventually, psychological over-grooming becomes habitual if the source of the problem is not properly identified and addressed. It is very important not only to identify the cause, which is not always feasible, but to immediately treat the psychological over-grooming, since the later treated, the more difficult to avoid that it becomes permanent. Displacement behaviour is usually thought of as self-grooming, touching, or scratching, which is displayed when an animal has a conflict between two drives, such as the desire to approach an object, while at the same time being fearful of that object. Displacement behaviours are linked with high anxiety levels (Maestripieri, de D. et al., A modest proposal: displacement activities as an indicator of emotions in primates. Anim. Behav. 1992, Vol. 44, pages 967-979).

Accordingly, in a more particular embodiment of the invention, any of the compositions and edible products of the invention is used to treat and/or prevent a non-human animal stereotype selected form the group consisting of: vacuum chewing, bar biting and displacement behaviour. In a particular embodiment, the displacement behaviour is psychological over-grooming.

Among the animal abnormal behaviours, animal social instability is also relevant.

In the context of the present invention, the expressions "animal social instability", "animal sociability problems" and "low degree of animal sociability" are used indistinctively to refer to the inadaptation of the animal to environmental conditions. They are manifested by problems in the formation of groups or aggregation. Under social stability the formation of groups may be temporary (for breeding, hibernation, or migration) or permanent, wherein the aggregation may be associated with the search for food, protection against enemies, mutual heating, etc.

Animal social instability results in aggression. Social aggression in the case of pets and livestock animals can take a variety of forms, which may be expressed physically or communicated verbally or non-verbally. Among them, and without limitation, defensive aggression (fear-induced), dominance aggression, inter-male aggression, resident-intruder aggression, maternal aggression, species-specific aggression, sex-related aggression, territorial aggression, isolation-induced aggression, irritable aggression, and brain-stimulation-induced aggression (hypothalamus).

As stated above, physical aggressions between animals are typical abnormal behaviours due to sociability problems. Among said physical aggressions between non-human animals, and without limitation, are mounting behaviour, animal feather pecking and tail and ear biting.

In summary, low degree of animal sociability, expressed by certain abnormal behaviours, are inversely associated with good/balanced/equilibrated animal sociability.

Accordingly, in a particular embodiment, the compositions of the present invention are used to treat and/or prevent social instability. In a still more particular embodiment, the compositions of the present invention are used to treat and/or prevent social aggression and/or territorial aggression.

In a still more particular embodiment of the invention, the non-human animal social and/or territorial aggression is manifested at least by one of the forms selected from the group consisting of: mounting behaviour, animal feather pecking, cannibalism and tail and ear biting.

In the context of the present invention, the expressions "mounting behaviour" and "bullying" are used indistinctively. Said expressions describe the situation when a male animal mounts another male animal. This is typical, without limitation, in unfamiliar male calves housed in a group, which is usually classified as a social aggression or a territorial behaviour in order to establish a social dominance and a hierarchy in a housed herd. The incidence of mounting behaviour implies a reduction in the time that the animal dedicates to chew, which involves an increased predisposition to suffer ruminal acidosis.

In the present invention, the expression "ruminal acidosis" is to be understood as an abnormal increase in the acidity of the body's fluids, caused either by accumulation of acids or by depletion of bicarbonates.

By the expression "feather pecking" is to be understood the situation wherein one bird repeatedly pecks another. Feather pecking is usually followed by pecking the body, toes, tail and especially the vent area.

Feather pecking can end up in serious bleeding and trauma of the skin, and eventually induce cannibalism and death. Outbreaks of feather pecking and cannibalism can occur in all types of housing systems including, without limitation, cages, floor pens, aviaries, outdoor, free-large flocks. Outbreaks also occur among different types of poultry, i.e., and without limitation, hens, chickens, ducks, turkeys, pheasants, quails and ostriches. Feather pecking and cannibalism are caused by a combination of genetic and environment factors, related to dominance relationships and formation of the dominance hierarchy.

The current solution to treat and/or prevent feather pecking is resorting to beak-trimming. Break trimming consists in the partial removal of the beak of poultry, especially turkeys although it may also be performed on quail and ducks. Consequently, the effective and non-aggressive way to treat and/or prevent feather pecking by using the compositions of the present invention, represents an important goal for poultry industry.

In the present invention, the expression "tail and ear biting" refers to an abnormal behaviour characterized by one pig's dental manipulation of another pig's tail and pig's ear, respectively. Tail and ear biting can become obsessive behaviours very quickly. The causes of tail and ear biting are difficult to identify. It has multifactorial origin, and among the described risk factors are lack of straw or other environmental enrichment conditions present in intensive systems, which increase competition, frustration and irritability between pigs. Normally, tail and ear biting are sporadic, making outbreaks difficult to predict and understand. Therefore, its prevention and/or treatment are essential.

In another particular embodiment, the compositions of the present invention when used to prevent and/or treat non-human animal abnormal behaviours, facilitates animal's manipulation. As described above, the animal abnormal behaviours hinder the animals' handling by the farmer and/or veterinarian. By treating and/or preventing non-human animal abnormal behaviours, the animal social aggression is reduced. As a consequence, animals are less obsessed with a specific behaviour and do not feel bothered or inconvenienced when there is an intervention by the farmer or veterinarian. Frequently, the presence of non-human animal abnormal behaviours, and especially social aggression, results in the physical damage to the animal. By treating and/or preventing them, the animal pain and discomfort is reduced, and with that its irritability. Consequently, animals are more lively, interactive and curious, and do not respond with fear to the human presence, improving their handling.

Non-human animal abnormal behaviour as well as social instability results in huge economic losses in the animal product industry. The main causes therefore being, without limitation, reduced weight gain, high animal mortality rate, culling and carcass condemnation and increased on-farm veterinary treatment.

Consequently, another advantage of the compositions or the edible products of the present invention is that when used for the prevention and/or treatment of non-human animal abnormal behaviour, such as bullying, cannibalism and feather pecking, it reduces or avoids important economic losses indirectly related to said abnormal behaviours. In general, the non-human abnormal behaviours spread quickly throughout the animals and are very difficult to control and reverse. For instance, feather pecking is a common cause of mortality in pheasant's farm because they are animals with competitive and territorial behaviour and with important dominant hierarchy, such as the pheasants.

Another abnormal behaviour related with physical aggression between non-human animals, which also represents significant economic costs, is tail and ear biting in intensive pig production systems. Obsessive tail and ear biters spend much more time biting tails or ears. Those animals who become obsessive tail or ear biters' are more likely to undergo a growth deficiency during earlier period and may therefore be smaller than expected. As for bitten animals, they are usually more predisposed to be ill.

In particular, the value of the animals affected by feather pecking and cannibalism decreases due to the damaged meat and poor plumage, but also in extreme cases due to the eventually high animal mortality.

In the case of animal bullying, it implies bruised tissues as well as an increase dark cutting meat. Bruised tissues also look unsightly and are usually trimmed because they decompose and spoil rapidly (i.e. bloody meat is an ideal medium for growth of contaminating bacteria). Additionally, the final consumer does not accept bruised tissues due to organoleptic reasons.

In the context of the present invention, the expression "dark cutting meat" is to be understood, as a quality defect where the meat is darker and drier than normal, reducing its life of being fit for consumption.

Animal bullying also involves a decrease in fodder intake by sow, and consequently reduction in milk production, which has negative consequences in the growth and health of suckling piglets, with higher sensitivity to bacterial, viral or parasite infections. In the worst case, animal bullying implicates less maternal instinct in sow, and consequently crushing of piglets by mother sow. The reduction of fodder intake by the sow is also negative by itself because it involves that the time to appear the next estrus cycle (or sexual desire, comprising the recurring physiologic changes that are induced by reproductive hormones in most mammalian therian females) after lactation phase, is longer and with minor fertility.

Frequently, the animal meat for consumption when the animal suffers any type of abnormal behaviour, especially those described herein, the meat is characterized by an abnormal colour, consistency, and water holding capacity, making the meat dry and unattractive to consumers.

Consequently, another aspect of the present invention is the compositions or the edible products of the present invention for use in the prevention and/or treatment of non-human animal abnormal behaviour in order to increase the quality and/or quantity of the animal products. As it will be illustrated by way of examples, and stated above, the ingestion of any of the compositions or the edible products of the invention provides many advantages.

In a particular embodiment, the increase in the quality of the animal product is manifested by one of the forms selected from the group consisting of: reduction of dark cutting meat, and/or enhanced organoleptic properties of the animal meat for consumption, and/or lower exudation, and/or tenderer cooked meat, being in all cases more attractive to the final consumer.

The Examples below illustrate a particular embodiment where 0.056 % by weight of the mixture of dried solids gives rise to 300 ppm of steviol glycosides in the final fodder or edible product. In another particular embodiment, the proportion of dried parts is 0.56 % by weight in the final edible product. A proportion of 0.56 % gives rise to 3,000 ppm of steviol glycosides in the final fodder or edible product.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and particular embodiments described herein.

### EXAMPLES

### EXAMPLE 1A. Edible product to treat mounting behaviour (bullying) in beef production Friesian male calves

The aim of this assay was to demonstrate the effect of the invention in the treatment (reduction) of mounting behaviour (bullying), animals' sociability and manipulation by the farmer and veterinarian in Friesian male calves of a single herd.

A fodder commonly used for beef production of Friesian male calves was supplemented with the composition of the invention. In particular, 2.5 kg of a solid powder of a mixture of dried leaves and dried stems of *Stevia rebaudiana bertoni* plant from Paraguay (Amambay mountain chain) in a ratio of 30:70 w/w %, were incorporated in 1,000 kg of a fodder commonly used to feed them. The mixture of dried leaves and dried stems represented 0.25 % by weight of the resulting edible product, wherein the edible product, in all the Examples, was the mixture of the fodder and a solid powder of a mixture of dried leaves and dried stems.

The above edible product was given to 150 Friesian male calves housed in the same herd, with appropriate salubrity, handling and installation conditions. The average calves' weight was around 380 kg, and the average age was 9 months old. The calves were housed in a barn. The daily intake of the edible product by each calf was approximately 8 kg. Consequently, the daily intake of the composition of the invention by each calf was 20 g, which corresponds to 52.63 mg of composition of the invention per kg of animal weight and per day.

After 7 days feeding the Friesian male calves with the above edible product, the mounting behaviour, affecting the entire herd before the treatment, was significantly reduced by 80%.

### Example 1 B. Effect of the edible product of the invention in the treatment of mounting behaviour in different farms of beef production with different races of male calves

The aim of this assay was again to demonstrate the effect of the invention in the treatment of mounting behaviour of several herds of male calves, located in different farms.

Twenty different farms, with appropriate salubrity, handling and installation conditions, with 50 male calves per farm located in the region of Lleida (Spain) were treated of mounting behaviour with the edible product of the invention. The calves' breeds were Friesian, Limousin, Charolaise, Bruna (from Pyrenees), Holstein and Montbeliard. The male calves were fed with the commonly used fodder for beef production, supplemented with the composition of the invention. In particular, 3 kg of a solid powder of a mixture of dried leaves and dried stems in a ratio of 30:70 w/w % of *Stevia rebaudiana bertoni* from Paraguay (Amambay mountain chain), were incorporated in 1,000 kg of the commonly used fodder. The mixture of dried leaves and dried stems represented 0.30 % by weight of the resulting edible product.

The animals were between 7 and 10 months old when the mounting behaviour began. Their weight was around 300 and 416 kg. The animals were housed in a barn. The daily intake by each animal of the above edible product was 6.5 and 9 kg, depending on the age of the animal. Accordingly, the daily intake of said composition of the invention by each animal was 19.5 and 27 g, respectively, which correspond to 65 mg of composition of the invention per kg of animal weight and per day.

After 7 days of feeding with the above edible product, the mounting behaviour that initially affected the entire herd was reduced in an 80 %. In consequence, both animal handling and manipulation by the farmer were easier, and the risk of the farmer to be injured was also significantly reduced. Similarly, by reducing the mounting behaviour, the ruminal acidosis predisposition in said animals suffering from mounting behaviour, due to the reduction of the time dedicated to chew, was effectively prevented.

Additionally, the reduction of mounting behaviour with the edible product of the invention resulted in a reduction of bruised and dark cutting meat, and hence, and increase in the quality of the meat.

### Example 2: Effect of the edible product of the invention in the prevention of feather pecking and cannibalism and improvement of sociability in pheasant production

The aim of this assay was to demonstrate the effect of the invention in the prevention of feather pecking and cannibalism in a farm -with appropriate salubrity, handling and installation conditions- of pheasants, type of poultry animal with an extremely high incidence of this type of abnormal behaviour. The pheasants fattened used in this assay showed a high incidence of feather pecking and cannibalism, especially in the tail area, to such an extent that the weekly mortality rate due to said abnormal behaviours was on average 6 %, whereas the mortality rate of pheasant fattened without said abnormal behaviours was 2 %, considered to be the normal rate.

Ten thousand new born male and female pheasants, half males and half females, were fed with a fodder commonly used for pheasant's production supplemented with the composition of the invention. In particular, 1 kg of a solid powder of a mixture of dried leaves and dried stems in a ratio of 30:70 w/w % of *Stevia rebaudiana bertoni* from Paraguay (Amambay mountain chain), was incorporated in 1,000 kg of the fodder. The mixture of dried leaves and dried stems represented 0.1 % by weight of the resulting edible product.

The pheasants were fed with the above edible product since they were 1 day old until they were 180 days old, which was the end of the fattening phase. At the end of the assay, the pheasants' weight average was 1.2 kg.

The average daily intake of the edible product by each animal was 85 g. Hence, the average daily intake of the composition of the invention by each animal was 85 mg. It corresponds to 70.83 mg of composition of the invention per kg of animal weight and per day.

The pheasants fed with the above edible product showed a 60 % reduction in the incidence of feather pecking and cannibalism (which are known to be caused by dominance relationships), in comparison with those pheasants fattened only with the commonly used fodder, both health and fattened under the same farm conditions.

In conclusion, the addition of the dried solids of the invention in the commonly used fodder treated the incidence of feather pecking and cannibalism in pheasants, by moderating their strong dominance relationships, and it also reduced the mortality rate. The normal mortality rate in pheasant production is 2 %. The mortality rate in the treated animals before being treated with the above edible product was 6 %. After said treatment, the mortality rate was reduced to 2.18 %, hence, to normal values.

### Example 3. Effect of the edible product of the invention in the prevention of stereotypes during the lactating phase in sows

The aim of this assay was to demonstrate the effect of the invention to prevent the appearance of stereotypes, in particular vacuum chewing, bar-biting and noisy vocalizations in lactating sows in a farm with appropriate salubrity, handling and installation conditions.

A first group, (Group 1 or Control Group) comprising 15 sows (Landrace x Large White), 6 primiparous and 9 multiparous sows, was fed with their habitual fodder, commonly used for lactating sows.

A second group (Group 2 or Treatment Group) comprising 12 sows (Landrace x Large White), 6 primiparous and 6 multiparous sows were fed with the habitual fodder commonly used for lactating sows, the same as that used to feed the animals of Group 1, but supplemented with the composition of the invention. In particular, 560 g of a powder of solid dried leaves and dried stems of *Stevia rebaudiana bertoni* plant from Paraguay (Amambay mountain chain) in a ratio of 30:70 w/w %, were incorporated in 1,000 kg of fodder. The mixture of solid dried leaves and dried stems represented 0.056 % by weight of the resulting edible product.

Sows in both groups were individually kept in typical stalls systems for sows and its piglets. There was an individual fodder trough and an individual water trough with ad libitum water in each stall.

The animals of Group 2 were fed with the above edible product from the last week of pregnancy (week before birth) up to the lactating phase (28 days), when they were with their piglets, in total during 35 days.

During the assay the weight average of the sows was 230 Kg. The average daily intake of the above edible product by each animal was 6 kg. Hence, the average daily intake of the composition of the invention by each animal was 3.36 g, which corresponds to 14.61 mg of composition of the invention per kg of animal weight and per day.

The farmer and veterinarian observed the sows' behaviour during the 35 days of treatment. The sows of Group 2 showed fewer incidences of vacuum chewing, bar-biting and noisy vocalizations when a piglet wanted to suck, in comparison with those of Group 1 or Control Group. In particular, 65 % of sows in the Control Group had stereotypies. They spent more than 15 % of their time in performing stereotypies; whereas only 20% of the sows in Group 2 showed stereotypical behaviours, in which they spent around 15 % of their time.

In conclusion, the addition of the mixture of the dried leaves and dried stems into the fodder in a proportion of 0.056 % prevented the incidence of stereotypes in lactating sows in intensive pig production. Consequently, prevent the following negative consequences of the animal products, i.e. reduction of milk production, reduction in growth and immunity in piglets, less instinct maternal and minor fertility in the next estrus.

### Example 4: Effect of the edible product of the invention in the prevention of the ear and tail biting stereotype in growing and fattening phase in pig production

The aim of this assay was to demonstrate the effect of the invention in the prevention of tail and ear biting in growing and fattening phase. The pigs, both in the control and the treatment groups, had the same age, and were under the same housing, environment and handling conditions in each farm.

The assay was carried out in 4 different farms with correct salubrity, handling and installations. Each farm had 170 pigs in growing phase (42 days), since they were 29 days old up to 61 days old.

The fodder commonly used for pigs in growing phase was supplemented with the composition of the invention. In particular, 560 g of a solid powder of a mixture of dried leaves and dried stems of *Stevia rebaudiana bertoni* from Paraguay (Amambay mountain chain) in a ratio of 30:70 w/w % were incorporated into 1,000 kg of the usual fodder. The solid mixture of dried leaves and dried stems represented 0.056 % by weight of the resulting edible product.

The same piglets during their fattening phase, lasting 107 days, since they were 62 days up to 169 days old, were fed with the fodder commonly used for pigs in growing phase supplemented with the composition of the invention. In particular, 560 g of a powder of a solid mixture of dried leaves and stems of *Stevia rebaudiana bertoni* from Paraguay (Amambay mountain chain) in a ratio of 30:70 w/w % were incorporated into 1,000 kg of fodder. The solid mixture of dried leaves and stems represented 0.056 % by weight of the resulting edible product.

The average weight of the pigs in growing phase was 20 kg, and 107 kg during the fattening phase.

The average daily intake of the edible product by each animal during the growing phase was 400 g, and 2 kg during fattening phase. Hence, the average daily intake of the composition of the invention by each animal in the growing phase was 224 mg, and 1.12 g in the fattening phase, which corresponds to 11.2 mg per kg of animal weight and per day in the growing phase, and 10.47 mg per kg of animal weight and per day in the fattening phase.

On each farm, the treated animals were compared with those of a control group, comprising the same number of animals (170), of the same race and age, and under the same environmental conditions fed only with their usual feed, i.e. without the composition of the invention. The treated animals, during the growing and fattening phase showed 70 % fewer incidence of ear and tail biting with respect to the animals of the control group. Additionally, the treated animals were more stable and sociable compared to the control group, without obsessive pig biters and without competition and frustration between pigs. Also, piglets of control group were more sensitive in presence to the farmer or veterinarian in the installations or corrals, showing fear and taking longer time until being quiet.

When the farmer or veterinarian entered the installations and stall or even the aisle, between corrals on each side, the animals of the control group ran, jumped, mounted on other pigs, shouted, etc., in summary, showed an escape reaction. Said reactions were not observed in the animals after treatment.

In conclusion, the above edible product prevented the ear and tail biting stereotype in pig production, resulting in a more social and stable group. The edible product reduced frustration and competition between pigs in front of adverse stimuli inherent and unavoidable in intensive pig production. The edible product reduced or modulated the escape reaction of pigs in front of persons, which implies jumping, running, shouting, mounting behaviour inside the corral, etc. Consequently it prevented the negative consequences like bruised carcasses and facilitated the handling by the farmer and veterinarian.

### Example 5. Effect of the edible product to treat the stereotype of over-grooming in a European shorthair cat

The aim of this assay was to demonstrate the effect of the invention to treat an indoor European shorthair cat with psychological over-grooming behaviour.

Normally, a healthy cat spends between 10-30 % of its time to groom; grooming which, under normal circumstances always starts in the head and continues down towards the tail. The cleanest areas are the face, especially the mouth area, the neck, the chest, the shoulders and the back.

The European cat was a 4 years old healthy male, without infectious or allergic reactions, sterilized, vaccinated and dewormed cat, weighting 5 kg. The cat was living in a flat, exposed during approximately one week to environmental adverse stimuli due to intense removal of furniture.

Thereafter, once the adverse stimuli disappeared, the cat showed excessive over-grooming, especially licking its paunch in a very quick and obsessive way. The time that the cat spent grooming was over 30 %, and only focusing on the belly area.

The over-grooming persisted during one month, causing a hairless abdomen with a very irritated skin. Then, the humid diet commonly used for domestic European shorthair cats was supplemented with the composition of the invention in order to treat the psychological over-grooming. In particular, 500 mg of a solid powder of a mixture of dried leaves and dried stems of *Stevia rebaudiana bertoni* from Paraguay (Amambay mountain chain) in a ratio of 30:70 w/w %, were incorporated in 80 g of the cat's habitual humid diet. The mixture of the dried leaves and dried stems represented 0.625 % by weight of the resulting edible product.

The average daily intake of the above edible product was 80 g. Consequently, the daily intake of the composition of the invention was 500 mg, which corresponds to 100 mg per kg of animal weight and per day.

After 10 days of treatment with the above edible product, the over-grooming began to reduce. After 10 more days of treatment, the percentage of daily time dedicated to grooming became normal, below 10 %, as well as the pattern of grooming, with a normal pattern from the head down to the tail, with calm and slow movements. Eventually, the hair of his abdomen began to grow. In summary, the psychological over-grooming was reduced due to a reduction in the sensibility and the emotional reactivity resulting from different changes in the environment. Consequently, the cat was successfully treated with the edible product of the invention, as evidenced by the gradual reduction of the psychological over-grooming, resulting in the prevention of a vicious cycle, and also by avoiding the appearance of abraded skin and serious infections.

### BIBLIOGRAPHIC REFERENCES

da Costa, M.J.R.P. et al. Strategies to promote farm animal welfare in Latin America and their effects on carcass and meat quality traits. Meet Sci. 2012, Vol. 92, No. 3, pages 221-6.
Veissier, I. et al. Animal welfare: A result of animal background and perception of its environment. Animal frontiers. 2012, Vol. 2, No. 3, pages 7-15.
Langley, R.L. and Morrow W.E. Livestock handling-minimizing worker injuries. J. Agromedicine. 2010, Vol. 15, No. 3, pages 226-35.
Beaumont, C. et al. The European experience in poultry welfare-A decade ahead. Poultry Science, 2012, Vol. 89, pages, 825-31.
Shiozaki, K. et al. Inhibitory effects of hot water extract of the Stevia stem on the contractile response of the smooth muscle of the guinea pig ileum. Biosci. 8iotechnol. Biochem. 2006, Vol. 70, pages 489-494.
Matera, S. et al. Sedative and antispasmodic effects of Stevia rebaudiana and non-competitive inhibition of intestinal contractility by stevioside. Pharmacology on line. 2012, Vol. 2, No. 1, pages 1-8.
Maestripieri, de D. et al. A modest proposal: displacement activities as an indicator of emotions in primates. Anim. Behav. 1992, Vol. 44, pages 967-979.

## Claims

1. A composition based on a dried part of *Stevia rebaudiana bertoni* plant for use in the prevention and/or treatment of at least one animal abnormal behaviour.

2. The composition according to claim 1, wherein the dried part of *Stevia rebaudiana bertoni* is selected from the group consisting of: dried stems, dried leaves and a mixture of dried leaves and dried stems.

3. The composition according to claim 2, wherein said composition is a solid powder, and wherein when the dried part of *Stevia rebaudiana bertoni* is a mixture of dried leaves and dried stems, the ratio of dried leaves and dried stems is comprised between 25:75 and 35:65 w/w %, preferably between 30:70 w/w %.

4. The composition according to any of the previous claims, wherein the animal is a non-human animal.

5. The composition according to claim 4, wherein the non-human animal is selected from the group consisting of: livestock animal and companion animal.

6. The composition according to claim 5, wherein the non-human animal is selected from the group consisting of: chicken, beef, pig, sheep, cow, hen, horse, donkey, pony, turkey, rabbit, quail, partridge, goat, pheasant, ostrich, duck, dog, cat, an exotic domestic animal and a zoo animal.

7. The composition according to any of the previous claims, wherein the at least one animal abnormal behaviour is a stereotype.

8. The composition according to claim 7, wherein the animal is a non-human animal and the stereotype is selected form the group consisting of: vacuum chewing, bar biting and displacement behaviour, preferably psychological over-grooming.

9. The composition according to any of the claims 1-6, wherein the at least one abnormal behaviour is social instability, preferably social aggression and/or territorial aggression.

10. The composition according to claim 9, wherein the animal is a non-human animal and the social instability is manifested at least by one of the forms selected from the group consisting of: mounting behaviour, animal feather pecking, cannibalism and tail and ear biting.

11. The composition according to any of the claims 4-6, 8 and 10, wherein the prevention and/or treatment of the at least one non-human animal abnormal behaviour facilitates animal manipulation.

12. The composition according to any of the claims 4-6, 8, 10 and 11, wherein the prevention and/or treatment of the at least one non-human animal abnormal behaviour increases the quality of the animal product.

13. The composition according to claim 12, wherein the increase in the quality of the animal product is manifested by one of the forms selected from the group consisting of: reduction and/or prevention of dark cutting meat, enhanced organoleptic properties of the animal meat for consumption, lower meat exudation, and tenderer cooked meat.

14. An edible product comprising the composition as defined in any of the previous claims and at least one edible acceptable ingredient.

15. The edible product according to claim 14, wherein the edible product is an animal fodder.
